# EUROPEAN PATENT APPLICATION

(11) **EP 2 016 831 A1**
(43) Date of publication of application: **21.01.2009**
(21) Application number: 07741431.6
(22) Date of filing: 11.04.2007
(51) Int. Cl.: A23C 3/00, A61K 35/14, B01D 19/00

(54) **METHOD OF GAS TREATMENT**

(30) Priority: 11.04.2006 JP 2006109094
(71) Applicant: Wingturf Co., Ltd., Tokyo 100-6510 (JP); Hashiba, Tomohiko, Tokyo 100-6510 (JP)
(72) Inventor: HASHIBA, Tomohiko, Chiyoda-ku Tokyo 10006510 (JP)
(74) Representative: Claessen, Rolf
(86) International application number: PCT/JP2007/057995
(87) International publication number: WO 2007/117024

(57) **Abstract**

The prevent invention provides a novel gas treatment method for a fluid, and a method for producing a milk using the same, which is industrially extremely advantageous in terms of cost and efficiency.

A gas treatment method for a fluid, comprises, while discharging a fluid from a fluid discharge port 161 of a two-fluid nozzle 160, crushing the discharge flow into fine droplets by a gas stream from a gas injection port 162, and then allowing the droplets to strike against a flow prevention member 190 and thereby agglomerate, so as to perform a gas treatment (gas addition, gas replacement, deaeration, or sterilization (disinfection)). According to the method for producing a milk, nitrogen is used as the gas stream, whereby homogenization and nitrogen replacement of dissolved oxygen can be performed in one and the same process.

## Description

### TECHNICAL FIELD

The invention relates to a gas treatment method for fluids including blood, beverages, liquid foods, medicines, or the like.

### BACKGROUND ART

In the manufacturing of various products, a gas treatment process is involved.

For example, in the production of fluid foods including beverages, liquid foods, or the like, gases dissolved in such foods are subjected to replacement for the purpose of preventing deterioration of the flavor. In the production of alcoholic beverages, oxygen is occasionally added in order to enhance the flavor.

Also, in the production of milk beverages including cow's milk, and the like, a treatment to reduce the dissolved oxygen concentration in raw milk is sometimes performed for the purpose of preventing deterioration of the flavor. For example, Patent Document 1 and Patent Document 2 disclose a method for producing a milk, the method including subjecting raw milk to replacement with a nitrogen gas or like inert gas prior to heat sterilization to reduce the amount of dissolved oxygen, thereby suppressing the generation of sulfides (sulfur compounds) upon heat sterilization. Patent Document 3 discloses a method including agitating raw milk stored in a silo while passing a nitrogen gas therethrough to reduce the dissolved oxygen concentration in the raw milk, thereby suppressing the growth of harmful microorganisms.

Further, in the production of milks, fat globules contained in the raw fresh milk are fined, and then homogenized for the purpose of preventing the separation of fat. Homogenization is usually performed by applying pressure to the raw milk to break the milk fat.

Further, in the manufacturing of various products, a sterilization treatment process is involved.

For example, as described in Patent Document 2, a method for manufacturing a milk beverage includes replacement with a nitrogen gas, followed by heat sterilization. In the field of medical technology, heat sterilization is occasionally applied to blood, medicine, and the like (e.g., Patent Document 4). None of such conventional heat sterilization methods involves use of a gas.
[Patent Document 1] Japanese Patent No. 3083798
[Patent Document 2] Japanese Patent No. 3091752
[Patent Document 3] JP-A-05-49395
[Patent Document 4] JP-A-06-319463

### DISCLOSURE OF THE INVENTION

Prior art has problems in that the above gas treatment methods and heat sterilization methods are time-consuming and inefficient.

Further, milks are produced based on batch processing including the above treatment processes each performed separately and independently, which thus is problematic in terms of the time required for the production of milks as well as the cost therefor.

Therefore, the object of the present invention is to provide a novel and efficient gas treatment method.

A further object of the invention is to provide a method for producing a milk, which allows the above plurality of processes to be performed at once.

The present inventors conducted extensive research to solve the above problems. As a result, they found that spraying fresh milk from a two-fluid nozzle enables homogenization of the fresh milk, and that such dispersed state provides extremely high reactivity, in which state nitrogen replacement of dissolved oxygen present in the raw milk and sterilization can be performed extremely efficiently; they accordingly accomplished the present invention. Specifically, the present invention relates to the following (1) to (10).
(1) A gas treatment method for a fluid, comprising, while discharging a fluid from a fluid discharge port, crushing the discharge flow into fine droplets by a gas stream, and then allowing the droplets to agglomerate.
(2) A gas treatment method according to (1), wherein the gas stream contains oxygen to add oxygen to the fluid.
(3) A gas treatment method according to (1), wherein the gas stream contains nitrogen to replace a gas dissolved in the fluid with nitrogen.
(4) A gas treatment method according to any one of (1) to (3), wherein the gas stream contains superheated steam to sterilize the fluid.
(5) A gas treatment method according to any one of (1) to (4), wherein the fluid is blood, a beverage, a liquid food, or a medicine.
(6) A gas treatment method according to (1), wherein the fluid is raw milk, and the gas stream contains a nitrogen gas.
(7) A gas treatment method according to (1) or (6), wherein the fluid is raw milk, and the gas stream contains superheated steam.
(8) A method for producing a milk, comprising, while discharging raw milk from a fluid discharge port, crushing the discharge flow into fine droplets by a gas containing nitrogen, and then allowing the droplets to agglomerate, so as to simultaneously perform homogenization of the raw milk and nitrogen replacement of dissolved oxygen.
(9) A method for producing a milk, comprising, while discharging raw milk from a fluid discharge port, crushing the discharge flow into fine droplets by a gas containing overheated stream, and then allowing the droplets to agglomerate, so as to simultaneously perform homogenization of the raw milk and sterilization.
(10) A method for producing a milk, comprising, while discharging raw milk from a fluid discharge port, crushing the discharge flow into fine droplets by a gas containing nitrogen and overheated stream, and then allowing the droplets to agglomerate, so as to simultaneously perform homogenization of the raw milk, nitrogen replacement of dissolved oxygen, and sterilization.

In the method for producing a milk of the present invention, raw milk is a liquid containing fresh milk and components from fresh milk (especially fat). Milks encompass cow's milk, partially defatted milk, defatted milk, processed milk, a milk beverage, and the like produced from the raw milk as a raw material through various processes (homogenization, replacement of dissolved oxygen, sterilization, or the like).

### EFFECT OF THE INVENTION

According to the gas treatment method of the present invention, while discharging blood, a beverage, a liquid food, a medicine, raw milk, or a like fluid from a fluid discharge port, the discharge flow is crushed into fine droplets by a gas stream, and the droplets are then allowed to agglomerate. This makes it possible to subject the fluid to efficient gas addition, gas replacement, deaeration, or sterilization (disinfection).

Further, according to the method for producing a milk of the present invention, nitrogen replacement and/or sterilization of dissolved oxygen can be performed simultaneously with homogenization in one and the same process, thereby enabling efficient production of milks.

### BRIEF DESCRIPTION OF DRAWINGS

Fig. 1 shows a schematic diagram of one embodiment of processing equipment for homogenization of a fluid used in the production method of the invention;
Fig. 2 shows (a) a plan view of a two-fluid nozzle according to one embodiment, and (b) a sectional view of a two-fluid nozzle according to one embodiment;
Fig. 3 shows a front view of a two-fluid nozzle according to one embodiment;
Fig. 4 shows a block diagram of a constitutional example of a control device;
Fig. 5 shows the measurement results of the Examples; and
Fig. 6 shows the measurement results of the Examples.

### DESCRIPTION OF REFERENCE NUMERALS

- 100: Production Equipment
- 110: Raw Material Supply System
- 111: Raw Material Tub
- 112: Raw material Fluid
- 124: Processed Liquid
- 151: Fluid Supply Port
- 152: Gas Supply Port
- 160: Two-Fluid Nozzle
- 161: Fluid Discharge Port
- 162: Gas Injection Port
- 180: Control Device
- 190: Flow Prevention Member

### BEST MODE FOR CARRYING OUT THE INVENTION

The present invention is explained in detail hereinafter with reference to a preferable example of equipment used for the gas treatment for a fluid according to the present invention.

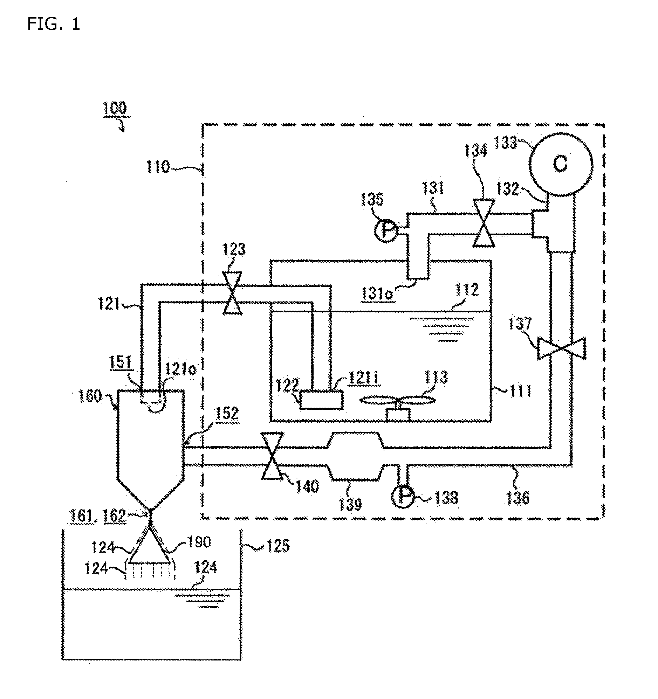

Fig. 1 shows a block diagram of one embodiment of processing equipment used for the gas treatment method for a fluid according to the present invention.

Production equipment 100 comprises a raw material supply system 110, a two-fluid nozzle 160, and a flow prevention member (baffle board) 190.

The raw material supply system 110 comprises a raw material tub 111. The raw material tub 111 is a closable, pressure-resistant container that is closed after fresh milk or a like fluid 112 is poured thereinto. The raw material tub 111 is provided at the bottom with an agitation device 113 comprising rotor blades for agitating a fluid 112.

Through the side wall of the raw material tub 111, a raw material supply pipe 121 is connected to the raw material tub 111. The inlet 121i of the raw material supply pipe 121 is located near the inner bottom surface of the raw material tub 111. The inlet 121i of the raw material supply pipe 121 has attached thereto a strainer 122.

The outlet 121o of the raw material supply pipe 121 is connected to a fluid supply port 151 of the two-fluid nozzle 160. In the midpoint of the raw material supply pipe 121, an electromagnetic variable throttle valve 123 for regulating a flow rate is disposed.

Through the ceiling wall of the raw material tub 111, a pressure pipe 131 is connected to the raw material tub 111. The outlet 131o of the pressure pipe 131 is located near the ceiling surface of the raw material tub 111.

The pressure pipe 131 is a pipe for introducing a compressed gas into the headspace inside the raw material tub 111 (space that exists above a fluid 112). The uppermost-stream end of the pressure pipe 131 is connected to a compressed-gas discharge port of a compressor 133 through a branch pipe 132. Midway along the pressure pipe 131 are disposed an electromagnetic valve 134 and an atmospheric pressure sensor 135 for detecting the atmospheric pressure in the headspace of the raw material tub 111.

A gas supply pipe 136 is connected to a gas supply port 152 of the two-fluid nozzle 160. The uppermost-stream end of the gas supply pipe 136 is connected to an exhaust port of the compressor 133 through the branch pipe 132. That is, the branch pipe 132 has two branches, and one outlet of the branch pipe 132 is connected with the pressure pipe 131, while the other outlet is connected with the gas supply pipe 136. Along the gas supply pipe 136 are provided, from the upper stream side to the lower stream side, an electromagnetic valve 137, an atmospheric pressure sensor 138, a compressed gas reservoir 139, and a pressure regulating valve 140 in this order. The atmospheric pressure sensor 138 is a sensor for detecting the atmospheric pressure in the compressed gas reservoir 139.

The compressor 133 is for generating a compressed gas. A compressed gas discharged from the compressor 133 is distributed to the pressure pipe 131 and the gas supply pipe 136 through the branch pipe 132. The gas supply pipe 136 is a pipe for introducing the compressed gas into the two-fluid nozzle 160. The compressed gas supplied to the gas supply pipe 136 is stored in the compressed gas reservoir 139, adjusted to a predetermined pressure, and then introduced into the two-fluid nozzle 160.

The two-fluid nozzle 160 is provided at the front end portion with a fluid discharge port 161 communicated to the fluid supply port 151 and also with a gas injection port 162 communicated to the gas supply port 152. A gas injection port 162 is formed around the fluid discharge port 161.

Below and near the two-fluid nozzle 160, a flow prevention member 190 made of stainless steel is provided. The flow prevention member 190 is a member having a conical shape that tapers upward. The tip (upper end) thereof is opposed to the fluid discharge port 161 of the two-fluid nozzle 160. The two-fluid nozzle 160 and the flow prevention member 190 are accommodated together in a not-illustrated right circular tube. They are connected to the inner wall of the right circular tube, and thereby held.

The fluid 112 supplied to the fluid supply port 151 of the two-fluid nozzle 160 is discharged from the fluid discharge port 161. In front of the two-fluid nozzle 160 (below the nozzle in the figure) is formed a high-speed vortex injected from the gas injection port 162. A discharged fluid 112 is crushed into fine particles (atomized) by this high-speed vortex. The flow strikes against the flow prevention member 190 immediately after the crush. As a result, the crushed flow undergoes reagglomeration (reagglomeration of atomized droplets) immediately after the crush, giving a processed liquid 124 in which the fluid is in a uniform state. The liquid 124 that has undergone reagglomeration on the flow prevention member 190 flows down along the surface of the flow prevention member 190. The liquid 124 that flows off the lower end of the flow prevention member 190 is collected in a product container 125.

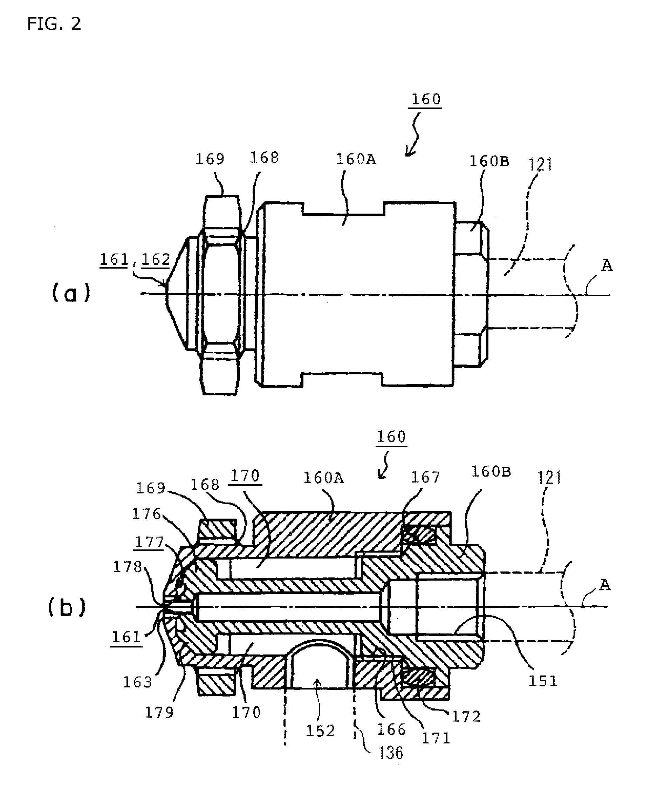

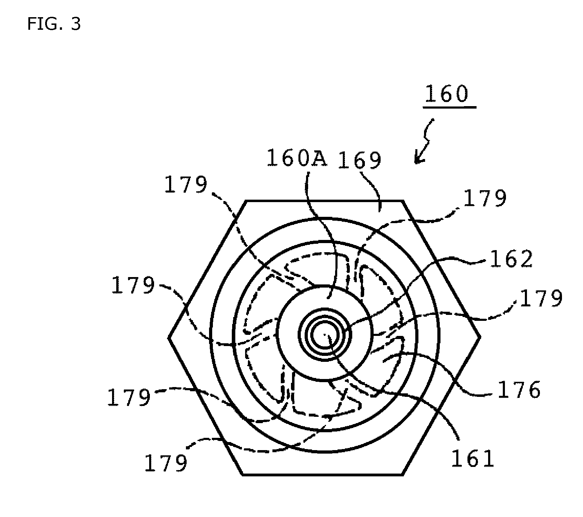

Next, the structure of the two-fluid nozzle 160 is explained with reference to Figs. 2 and 3. Fig. 2 (a) shows a plan view of a nozzle, and Fig. 2 (b) shows a sectional view of a nozzle. Fig. 3 shows a front view of a nozzle.

A two-fluid nozzle 160 has a structure such that an approximately cylindrical core 160B is inserted and screwed into an approximately cylindrical hollow casing 160A. The casing 160A is produced by mechanically processing a metallic material such as stainless steel, brass, or the like, and provided at the front end portion thereof with an opening hole 163. The opening hole 163 is concentric with the central axis A of the two-fluid nozzle 160 and has a circular cross section. The opening hole 163 forms the outer outline of a gas injection port 162. A gas supply port 152 is formed in the side of the casing 160A, in such a manner that the gas supply port 152 has an axis perpendicular to the central axis A of the two-fluid nozzle 160. A female thread is formed in the inner surface of the gas supply port 152, so that the gas supply pipe 136 can be screwed and engaged thereinto. A female thread 166 is formed at the proximal end inside the casing 160A, and a step portion 167 is formed at a portion closer to the proximal end, where the inside diameter is slightly larger. A male thread 168 is formed in the external surface at the front end portion of the casing 160A, so that a fixing nut 169 for attaching the two-fluid nozzle 160 can be screwed thereonto.

The core 160B is produced by mechanically processing a metallic material same as or different from one used for the casing 160 A, and the inside is hollowed along with the central axis A. The outer diameter thereof has a dimension such that the core closely fits within the cavity of the hollow casing 160A. Its outer diameter near the approximate center in the longitudinal direction is formed slightly thinner, so that a circular tubular space 170 exists between the core and the inner surface of the casing 160A. The space 170 is communicated with the gas supply port 152 provided in the casing 160A. A male thread 171 is formed at the periphery of the core 160B, slightly before the proximal end. The male thread 171 screws with the above-described female thread 166 to fix the core 160B in the casing 160A. The portion more proximal than the thread 171 has a slightly larger diameter, and holds an O-ring seal 172 between the same and the above-described step portion 167 to ensure the airtightness of the above-described space 170. A fluid supply port 151 is formed at the proximal end of the core 160B. A female thread is formed at the inner periphery of the liquid supply port 151, and the front end portion of a confluence pipe 135 screws and engages thereinto. At the front end portion of the core 160B, a fluid discharge port 161 communicated through the internal hollow space from the fluid supply port 151 is opened. The approximately conical, expanded portion around the fluid discharge port 161 serves as a spiral-forming member 176. A vortex chamber 177 is formed between the front end surface of the spiral-forming member 176 and the inner front end surface of the casing 160A. A space exists between the front end surface 178 of the core 160B, which forms the vortex chamber 177, and the above-mentioned opening hole 163 of the casing 160A; this serves as the gas injection port 162.

Referring to the front view of the two-fluid nozzle 160 shown in Fig. 3, a circular fluid discharge port 161 is located at the center, and the cyclic gas injection port 162 is located at the periphery thereof. The gas injection port 162 is communicated with a plurality of swirl slots 179. The swirl slots 179 are formed on the conical surface of the spiral-forming member 176 located inside the casing 160A, and extend spirally.

The compressed gas supplied from the gas supply port 152 passes through the space 170, and is compressed when passing through the swirl slots 179 with a small cross-sectional area formed in the spiral-forming member 176, whereby the compressed gas becomes a high-speed gas stream. The high-speed gas stream turns into a spiral swirling gas stream inside the vortex chamber 177, and is injected from the narrowed, circular gas injection port 162, forming a high-speed vortex gas stream in front of the two-fluid nozzle 160. This vortex is formed in the shape of a cone that tapes off focusing on the front position adjoining to the front end portion of the casing 160A.

An unmixed raw material fluid 112 sent out from the raw material tub 111 is supplied to the fluid supply port 151 through the raw material supply pipe 121. The fluid 112 supplied to the fluid supply port 151 is discharged from the fluid discharge port 161 through the hollow portion of the core 160B. The fluid is then crushed into fine particles by the high-speed vortex gas stream injected from the gas injection port 162, forcibly mixed with the rotation of the vortex, and released in the atomized state towards the front of the two-fluid nozzle 160 as a uniform mixture of fine particles. In the illustrated example, the inside diameter of the fluid discharge port 161 is slightly smaller than the inside diameter of the bore of the core 160B; however, when there is a potential for clogging, the inside diameter of the fluid discharge port 161 is preferably the same as the inside diameter of the bore.

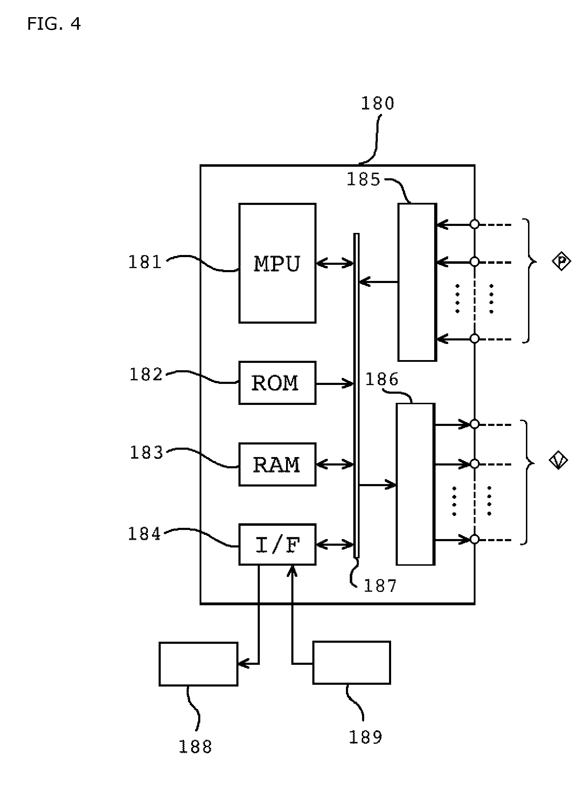

The production equipment 100 is controlled by a control device 180 shown in Fig. 4. The control device 180 incorporates an MPU 181, a ROM 182, a RAM 183, an interface unit 184, an A/D converter 185, and a drive unit 186. These are connected to one another through a bus line 187. The ROM 182 stores a program executed by the MPU 181. The RAM 183 is used as the workspace, or the like, upon execution of a program by the MPU 181. A display 188 such as a CRT is connected to the output port of the interface unit 184, and an input device 189 such as a keyboard is connected to the input port.

The input of the A/D converter 185 is connected with the atmospheric pressure sensors 135 and 138 of the production equipment 100, and the analog values of the air pressure detected by these sensors are converted into digital values. The digital values of the air pressure obtained by the conversion are read by the MPU 181 via the bus line 187.

The output of the drive unit 186 is connected to electromagnetism drive valves 123, 134, 137, and 140 of the production equipment 100. According to the command from the MPU 181, the drive unit 186 adjusts the current for these electromagnetism drives, and switches between ON and OFF.

For operating the production equipment 100, an operator puts a fluid into a raw material tub 111, and firmly closes the lid of the raw material tub 111. Subsequently, a command to start mixing is sent from an input device 189. Once this command is received, the MPU 181 sends a command to the drive unit 186 to open the electromagnetic valve 134. At the same time, the MPU 181 supervises the output from the atmospheric pressure sensor 135 through the A/D converter 185, and waits until the headspace of the raw material tub 111 is filled with a compressed gas from a compressor 133 and reaches a predetermined pressure. In this initial state, other electromagnetic valves of the production equipment 100 are closed. Once an atmospheric pressure sensor 135 of the raw material tub 111 confirms that the air pressure in the tank has increased to a predetermined level, the MPU 181 closes the electromagnetic valve 134. Subsequently, the electromagnetic valve 137 is opened. Accordingly, the compressed gas is supplied into a compressed gas reservoir 139.

Once the internal pressure of the compressed gas reservoir 139 has increased to a predetermined level, the MPU 181 judges that conditions are right to start the treatment, and opens the pressure regulating valve 140. Then, the compressed gas is supplied from the compressed gas reservoir 139 to a gas supply port 152 of a two-fluid nozzle 160, and a high-speed vortex gas stream is injected from a gas injection port 162 at the front end portion of the two-fluid nozzle 160. Next, the MPU 181 opens an electromagnetic variable throttle valve 123 to a predetermined degree. Accordingly, the raw material fluid 112 stored in the raw material tub 111 is supplied to a fluid supply port 151 of the two-fluid nozzle 160 through the raw material supply pipe 121, and discharged from the fluid discharge port 161 at the front end portion of the two-fluid nozzle 160. The raw material fluid 112 discharged from the two-fluid nozzle 160 is crushed into fine particles by the high-speed air vortex already formed in the discharge direction, and, with the vortex flow, released into a product container 125 in the state that the components of the raw material fluid 112 (fluid) are uniformly mixed.

As the above-described treatment proceeds, the level of the raw material fluid 112 in the raw material tub 111 is lowered. Accordingly, the volume of headspace of the raw material tub 111 increases, while the atmospheric pressure decreases. The pressure is always detected by the atmospheric pressure sensor 135, and obtained values are transmitted to the MPU 181. The MPU 181 always supervises the values detected by the atmospheric pressure sensor 135. When the value falls below a proper value, the electromagnetic valve 134 of the raw material tub 111 is switched to the open state for an appropriate time to thereby maintain the atmospheric pressure in the raw material tub 111 at a predetermined proper value. The pressure of the compressed gas inside the compressed gas reservoir 139 is also maintained at a proper value by the MPU 181 controlling the electromagnetic valve 137.

According to the processing equipment 100 of the embodiment, due to the operation explained above, a fluid (raw material fluid 112) is subjected to a gas treatment (gas addition, gas replacement, deaeration, or sterilization (disinfection)) in the state that the components of the fluid are uniformly mixed, giving a processed liquid 124, which is then accommodated in the product container 125 and collected.

Further, according to this processing equipment 100, the gas stream injected from the two-fluid nozzle 160 may be oxygen, whereby oxygen can be added to the fluid (or dissolved gas can be replaced with oxygen). The gas stream may also be an inert gas such as a nitrogen gas or carbon dioxide, whereby the dissolved oxygen and the like in the fluid can be replaced with the inert gas.

The gas stream injected from the two-fluid nozzle 160 may also be superheated steam (for example, 115°C to 200°C), hydrogen peroxide gas, or ozone, whereby the fluid can be sterilized in an atomized state with extremely high reactivity, and therefore, it becomes possible to complete sterilization efficiently within an extremely short time.

In the flow path in the two-fluid nozzle 160 to the fluid discharge port 161, a gas injection function may be provided for injecting a second gas (which may usually be the same as the gas used for crush) into the fluid in advance. As a result thereof, when the fluid is discharged from the fluid discharge port 161, finer crushing is possible due to diffusion of the injected gas, thereby achieving improved homogeneity.

Further, according to the processing equipment 100 of the embodiment, using raw milk as a fluid, a processed liquid 124 in which the moisture and fat globules in the fluid are uniformly mixed is produced, accommodated in the product container 125, and then collected. If the gas stream injected from the two-fluid nozzle 160 in that case is a nitrogen gas, dissolved oxygen can be replaced with nitrogen simultaneously with homogenization. Further, although the problem of foaming should be considered when employing a conventional nitrogen replacement method, this treatment method does not raise such a problem. If the gas stream injected from the two-fluid nozzle 160 is superheated steam, the fluid can be sterilized in an atomized state, and therefore, it becomes possible to complete sterilization efficiently within an extremely short time. [0058]

Fluid is not limited to the above example insofar the viscosity thereof allows the fluid to be fed with in a pipe by the difference in the pressure between the upper stream side and the lower stream side in the pipe.

### Examples

Hereafter, the prevent invention is explained in further detail through the Examples. In the following examples, as processing equipment (mixer), the processing equipment 100 explained above was used.

### (Dissolved oxygen concentration measurement and particle size measurement on samples treated with fresh milk and pure water)

### (1) Experimental Sample

Fresh milk (untreated) and pure water were used as samples.

### (2) Experimental Method:

### 1) Examination for Measurement of Dissolved Oxygen Concentration

The samples were each adjusted to a predetermined temperature (5°C, 10°C, 15°C, 20°C) in a thermobath (thermobath: THERMO MINDER SJ-10 (TAITEC), specified temperature range: 0 to 100°C, temperature accuracy: ±0.15 to 0.3°C), and then treated in a mixer (sample discharge pressure: 0.2 MPa, gas stream injection pressure: 0.5 MPa) as follows.

Nitrogen-gas treatment: treated in a mixer one to three times.

Oxygen-gas treatment: treated in a mixer one to three times.

Oxygen-gas treatment followed by nitrogen-gas treatment: samples treated in a mixer with an oxygen gas three times were further treated with a nitrogen gas.

### 2) Examination for Measurement of Change in Fat Globule Particle Size in Fresh Milk

Fresh milk diluted 1000 times with pure water was treated in a mixer one to three times.

### (3) Evaluation Method

After treatment of each sample, the dissolved oxygen concentration and the particle size were measured (cumulant method) using the following dissolved oxygen meter and light scattering photometer. The temperatures of the treated samples were also measured.

Dissolved-oxygen measurement: Digital dissolved oxygen meter DO-5509 (Fuso Co., Ltd.), measurement method; polarographic system (provided with a temperature sensor), measurement range; 0 to 20.0 mg/L, accuracy; ±0.4 mg/L

Particle size measurement: dynamic light scattering photometer DLS-7000 (Otsuka Electronics Co., Ltd.)

### (4) Evaluation Results

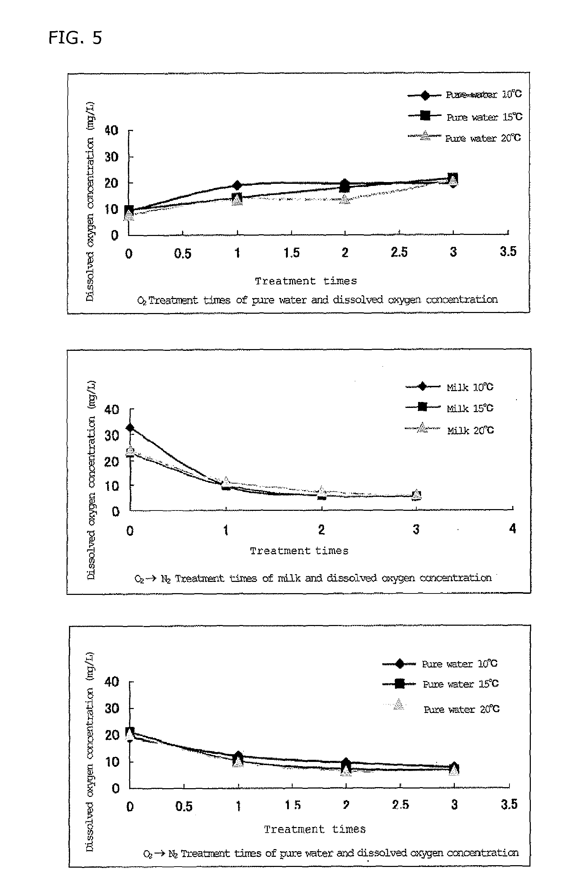

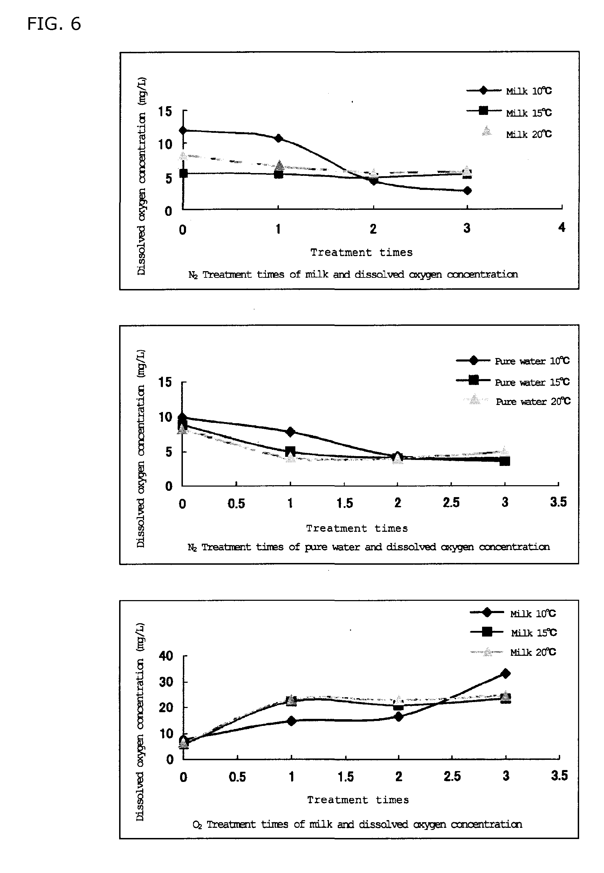

Table 1 shows dissolved oxygen concentration measurement values near 5°C and temperatures after treatment (measured temperature). Tables 2 to 4 show dissolved oxygen concentration measurement values and temperatures after treatment (measured temperature). The measurement results are shown in Figs. 5 and 6. Table 5 shows the average particle size in each sample (µm) and the proportion (%). Measured values of 20 mg/L or more (underlined) are shown as reference values. For a measured value of a sample untreated with O₂, a value measured in a sample untreated with N₂ was used (measured values in parentheses). For a measured value of a sample treated with O₂ → N₂, a value measured in a sample treated with O₂ three times was used (measured values in parentheses).

**[Table 1]**

| | Cow's milk | | | Pure water | | |
|---|---|---|---|---|---|---|
| | N₂ Treatment | O₂ Treatment | O₂→N₂ Treatment | N₂ Treatment | O₂ Treatment | O₂→N₂ Treatment |
| Untreated | 9.1 mg/L (6°C) | (9.1 mg/L) (6°C) | (34.5 mg/L) (7°C) | 11.2 mg/L (4°C) | (11.2 mg/L) (4°C) | (24.5 mg/L) (7°C) |
| Fi rst Treatment | 5.2 mg/L (7°C) | 38.3 mg/L (7°C) | 13.7 mg/L (7°C) | 6.4 mg/L (7°C) | 19.3 mg/L (6°C) | 15.0 mg/L (7°C) |
| Second Treatment | 4.9 mg/L (7°C) | 40.2 mg/L (7°C) | 7.3 mg/L (7°C) | 4.8 mg/L (7°C) | 23.2 mg/L (65°C) | 8.5 mg/L (7°C) |
| Third Treatment | 6.0 mg/L (7°C) | 34.5 mg/L (7°C) | 5.7 mg/L (7°C) | 5.0 mg/L (7°C) | 24.5 mg/L (7°C) | 6.2 mg/L (7°C) |

**[Table 2]**

| (Test near 10°C) | | | | | | |
|---|---|---|---|---|---|---|
| | Cow's milk | | | Pure water | | |
| | N₂ Treatment | O₂ Treatment | O₂→N₂ Treatment | N₂ Treatment | O₂ Treatment | O₂→N₂ Treatment |
| Untreated | 12.0 mg/L (6°C) | 7.6 mg/L (8°C) | (32.6 mg/L) (10°C) | 9.9 mg/L (9°C) | 9.3 mg/L (9°C) | (19.2 mg/L) (10°C) |
| Fi rst Treatment | 10.6 mg/L (8°C) | 14.5 mg/L (9°C) | 9.6 mg/L (10°C) | 7.7 mg/L (12°C) | 18.8 mg/L (10°C) | 12.1 mg/L (9°C) |
| Second Treatment | 4.1 mg/L (8°C) | 16.1 mg/L (9°C) | 5.8 mg/L (10°C) | 4.2 mg/L (9°C) | 19.3 mg/L (10°C) | 9.3 mg/L (9°C) |
| Third Treatment | 2.7 mg/L (8°C) | 32.6 mg/L (10°C) | 5.2 mg/L (10°C) | 3.8 mg/L (10°C) | 19.2 mg/L (10°C) | 7.4 mg/L (9°C) |

**[Table 3]**

| (Test near 15°C) | | | | | | |
|---|---|---|---|---|---|---|
| | Cow's milk | | | Pure water | | |
| | N₂ Treatment | O₂ Treatment | O₂→N₂ Treatment | N₂ Treatment | O₂ Treatment | O₂→N₂ Treatment |
| U ntreated | 5.6 mg/L (16°C) | 5.9 mg/L (15°C) | (22.9 mg/L) (16°C) | 8.8 mg/L (16°C) | 9.6 mg/L (16°C) | (21.1 mg/L) (16°C) |
| Fi rst Treatment | 5.4 mg/L (16°C) | 22.1 mg/L (16°C) | 9.6 mg/L (16°C) | 4.9 mg/L (16°C) | 14.0 mg/L (16°C) | 10.2 mg/L (16°C) |
| Second Treatment | 4.8 mg/L (16°C) | 20.5 mg/L (16°C) | 5.8 mg/L (16°C) | 3.9 mg/L (16°C) | 17.8 mg/L (16°C) | 6.9 mg/L (16°C) |
| Third Treatment | 5.2 mg/L (16°C) | 22.9 mg/L (16°C) | 5.2 mg/L (16°C) | 3.4 mg/L (16°C) | 21.1 mg/L (16°C) | 6.4 mg/L (16°C) |

**[Table 4]**

| (Test near 20°C) | | | | | | |
|---|---|---|---|---|---|---|
| | Cow's milk | | | Pure water | | |
| | N₂ Treatment | O₂ Treatment | O₂→N₂ Treatment | N₂ Treatment | O₂ Treatment | O₂→N₂ Treatment |
| U ntreated | 8.3 mg/L (20°C) | 6.5 mg/L (21°C) | (24.0 mg/L) (19°C) | 8.2 mg/L (20°C) | 7.9 mg/L (20°C) | (20.2 mg/L) (20°C) |
| Fi rst Treatment | 6.4 mg/L (20°C) | 22.7 mg/L (20°C) | 11.3 mg/L (19°C) | 4.1 mg/L (20°C) | 13.6 mg/L (20°C) | 9.8 mg/L (20°C) |
| Second Treatment | 5.5 mg/L (19°C) | 22.4 mg/L (10°C) | 7.3 mg/L (20°C) | 3.9 mg/L (20°C) | 13.6 mg/L (20°C) | 6.0 mg/L (20°C) |
| Third Treatment | 5.5 mg/L (19°C) | 24.0 mg/L (19°C) | 5.5 mg/L (20°C) | 4.8 mg/L (20°C) | 20.2 mg/L (20°C) | 6.0 mg/L (20°C) |

**[Table 5]**

| Sample | Peak 1 | Peak 2 | Peak 3 |
|---|---|---|---|
| Fresh Milk Untreated | 4.255 (97.4%) | 0.243 (2.6%) | - |
| Fresh Milk Treated Once | 3.822 (87.6%) | 0.169 (12.4%) | - |
| Fresh Milk Treated Twice | 3.221 (0.2%) | 0.162 (99.8%) | - |
| Fresh Milk Treated Three Times | 4.007 (0.8%) | 0.501 (0.2%) | 0.155 (99.0%) |

The above results show that when a fresh milk sample is treated in a mixer with a nitrogen gas, dissolved oxygen is efficiently replaced with nitrogen. Further, it was confirmed that a larger number of treatments lead to a higher proportion of peak with a small particle size.

## Claims

1. A gas treatment method for a fluid, comprising, while discharging a fluid from a fluid discharge port, crushing the discharge flow into fine droplets by a gas stream, and then allowing the droplets to agglomerate.

2. A gas treatment method according to claim 1, wherein the gas stream contains oxygen to add oxygen to the fluid.

3. A gas treatment method according to claim 1, wherein the gas stream contains nitrogen to replace a gas dissolved in the fluid with nitrogen.

4. A gas treatment method according to any one of claims 1 to 3, wherein the gas stream contains superheated steam to sterilize the fluid.

5. A gas treatment method according to any one of claims 1 to 4, wherein the fluid is blood, a beverage, a liquid food, or a medicine.

6. A gas treatment method according to claim 1, wherein the fluid is raw milk, and the gas stream contains a nitrogen gas.

7. A gas treatment method according to claim 1 or 6, wherein the fluid is raw milk, and the gas stream contains superheated steam.

8. A method for producing a milk, comprising, while discharging raw milk from a fluid discharge port, crushing the discharge flow into fine droplets by a gas containing nitrogen and/or superheated stream, and then allowing the droplets to agglomerate, so as to simultaneously perform homogenization of the raw milk, nitrogen replacement of dissolved oxygen, and/or sterilization.
